# EUROPÄISCHE PATENTSCHRIFT

(11) **EP 1 255 982 B1**
(45) Veröffentlichungstag und Bekanntmachung des Hinweises auf die Patenterteilung: **09.11.2005**
(21) Anmeldenummer: 00989805.7
(22) Anmeldetag: 28.11.2000
(51) Int. Cl.: G01N 27/414

(54) **MESSVERFAHREN UNTER EINSATZ MINDESTENS EINER BIOLOGISCHEN REZEPTORZELLE SOWIE EINE ZUR DURCHFÜHRUNG DES MESSVERFAHRENS GEEIGNETE VORRICHTUNG**
MEASURING METHOD BY MEANS OF ADDITION OF AT LEAST ONE BIOLOGICAL RECEPTOR CELL AND A SUITABLE DEVICE FOR CARRYING OUT SAID METHOD
PROCEDE DE MESURE AU MOYEN D'AU MOINS UNE CELLULE RECEPTRICE BIOLOGIQUE ET DISPOSITIF APPROPRIE POUR LA MISE EN OEUVRE DUDIT PROCEDE

(30) Priorität: 20.12.1999 DE 19961445
(43) Veröffentlichungstag der Anmeldung: 13.11.2002
(73) Patentinhaber: FORSCHUNGSZENTRUM JÜLICH GMBH, 52425 Jülich (DE)
(72) Erfinder: SCHÖNING, Michael, J., 52428 Jülich (DE); LÜTH, Hans, 52076 Aachen (DE); KAUPP, Ulrich, B., 52062 Aachen (DE); SCHÜTZ, Stefan, 35392 Gie en (DE); SCHROTH, Peter, 52064 Aachen (DE); THUST, Marion, 50939 Köln (DE)
(74) Vertreter: Naeven, Ralf
(86) Internationale Anmeldenummer: PCT/DE2000/004219
(87) Internationale Veröffentlichungsnummer: WO 2001/046692

(56) Entgegenhaltungen:
- DE-A- 3 735 702
- DE-A- 19 512 117
- US-A- 5 807 758
- SCHONING M J ET AL: "Miniaturization of potentiometric sensors using porous silicon microtechnology" ELECTROCHIMICA ACTA,GB,ELSEVIER SCIENCE PUBLISHERS, BARKING, Bd. 42, Nr. 20-22, 1997, Seiten 3185-3193, XP004086684 ISSN: 0013-4686
- THUST M ET AL: "POROUS SILICON AS A SUBSTRATE MATERIAL FOR POTENTIOMETRIC BIOSENSORS" MEASUREMENT SCIENCE AND TECHNOLOGY,GB,IOP PUBLISHING, BRISTOL, Bd. 7, Nr. 1, 1996, Seiten 26-29, XP000551467 ISSN: 0957-0233
- SCHROTH P ET AL: "INSECT-BASED BIOFETS WITH IMPROVED SIGNAL CHARACTERISTICS" BIOSENSORS & BIOELECTRONICS,ELSEVIER SCIENCE PUBLISHERS, BARKING,GB, Bd. 14, 15. März 1999 (1999-03-15), Seiten 303-308, XP000934104 ISSN: 0956-5663

## Beschreibung

Die Erfindung betrifft ein Messverfahren unter Einsatz mindestens einer biologischen Rezeptorzelle, bei dem die Reaktion der Rezeptorzelle auf Umgebungsänderungen mittels mindestens eines technischen Signalwandlers festgestellt und einer Auswertung zugeführt wird, sowie eine zur Durchführung des Messverfahrens geeignete Vorrichtung.

In der Biosensorik werden isolierte oder in Zellen oder Zellverbänden eingebaute biologische Rezeptorproteine (Enzymc, Antikörper, Rezeptoren) eingesetzt, um spezielle Substanzen nachzuweisen und/oder ihre Konzentration in der Umgebung der Rezeptorproteine zu bestimmen. Hierbei wird die hohe Selektivität der Rezeptorproteine sowie ihre hohe Empfindlichkeit genutzt. Rezeptorproteine, die beispielsweise in einer Zellmembran eingebaut sind, können im Falle des Kontakts mit einer zu detektierenden Substanz eine Reaktion in der Zelle bewirken. Das von nur einem Molekül angesprochene Rezeptorprotein kann einen oder mehrere ihm zugeordnete lonenkanäle öffnen und damit einen Transport von einigen zehntausend Ionen durch die Zellmembran hindurch induzieren. Hierdurch ist eine sogenannte biologische erste Signalverstärkungskaskade gegeben.

Die Reaktion der Zelle kann mit Signalwandern, wie Elektroden, Transistoren, Dioden, Fotozellen oder lichtadressierbaren Sensoren festgestellt und in einen mikroelektronischen Schaltkreis eingekoppclt werden. Die Schnittstellen zwischen halbleiterbasierenden, datenverarbeitenden Systemen und biologischen Systemen - angefangen von Biomolekülen bis hin zu intakten Zellen, Zcllsystemen sowie kompletten sensorischen Organen - sind dabei von großem Interesse. Verfahren zur signalübertragenden Ankopplung biologischer Systeme an Halbleiterstrukturen sind bekannt. So offenbart die DE 196 29 338 C1 ein Verfahren zur Bestimmung von Duftstoff-konzentrationen in der Umgebungsluft mittels Elektroantennogrammen. Dafür wird die Antenne einer Insektenart ausgewählt, die auf den nachzuweisenden Duftstoff ansprechende Rezeptorzellen aufweist. Aufgrund der naturgegebenen Anordnungen der Rezeptorzellen in den Insektenantennen werden in den Antennenstrukturen als Reaktion auf den zu detektierenden Duftstoff Multipolfelder erzeugt. Die Rezeptorzellen sind dabei so zueinander ausgerichtet, dass sich ihre temporären Multipolfelder additiv überlagern. Der in der Insektenantenne gegebene Zellverband sorgt somit für eine weitere Signalverstärkung (zweite Signalverstärkungskaskade). Das Multipolsignal kann ohne Zerstörung der Zellen und des Zellverbandes mittels Elektroantennogrammen festgestellt werden. Es ist allerdings kein gezieltes Auslesen von einzelnen Zellen möglich.

Bei dem Rezeptroden-Messaufbau (Rechnitz et al., Analytica, Chemica, Acta, 243 (1991), Seiten 157 - 166) werden die Aktionspotentiale einzelner Nervenfasern mittels externer Mikroelektroden abgegriffen. Der SCR(Single Cell Recording)-Messaufbau erlaubt es, eine Überlagerung von Rezeptorpotential und Aktionspotential eines einzelnen Sensillums zur registrieren. Sowohl beim Rezeptroden-Aufbau als auch bei der SCR-Technik werden mittels Mikroelektroden, zum Beispiel aus Glas, einzelne Sensillen, Nerven- oder Rezeptorzellen angestochen. Die Mikroelektroden müssen mit Mikromanipulatoren exakt positioniert werden. Dabei kommt es zu Verletzungen und Zerstörungen einzelner Zellbereiche, die für die technische Sensorik nicht gewünscht sind.

Aus der DE 195 12 117 A 1 sind ein Messverfahren und eine Vorrichtung der eingangs genannten Art bekannt. Die offenbarte Vorrichtung dient zur Messung oder Untersuchung physiologischer Parameter an biologischen Zellen oder von in einem Analyten enthaltenen chemischen Komponenten. In einem Substratmaterial befinden sich unterschiedliche Sensoren, die direkt unterschiedlichen funktionstauglichen Rezeptoren zugeordnet sind. Damit ist eine weitgehend rückwirkungsfreie Untersuchung der in einem umgebenden Analyten enthaltenen biologischen oder chemischen Komponenten möglich. Die Selektivität der Rezeptorzellen wird genutzt, um Konzentrationsänderungen in der Umgebung zu detektieren. Dabei kann als Messstruktur z. B. wenigstens ein Feldeffekttransistor vorgesehen sein, dessen Gate zum Kontakt mit den Rezeptorzellen freiliegt. Es können protonenspezifische Messungen vorgesehen sein. Die Messstruktur kann aber auch wenigstens einen Interdigitalkondensator mit paarig angeordneten Elektroden in Kamm- oder Mäanderstruktur aufweisen. Hiermit können Formveränderungen der Rezeptorzellen oder Impedanz- oder Kapazitätsänderungen der Zellmembran gemessen werden. Antikörper, die sich an den Rezeptorzellen anlagern, können ebenfalls nachgewiesen werden, da sie die Dielektrizitätskonstante im Bereich der Interdigitalstruktur verändern. Für die Messung können allerdings keine durch den Kontakt mit einer zu detektierenden Substanz erzeugten Änderungen innerhalb der Zelle ausgenutzt werden. Zudem ist diese bekannte Methode recht aufwendig.

Des weiteren ist es bekannt (P. Fromherz et al., Physical Review Letters, Vol. 71, Nr. 24, 1993, S. 4079 - 4083), dass Blutegelneuronen auf der aus SiO₂ bestehenden Gate-Oberfläche eines MOSFETs angesiedelt werden können (Neuron-Transistor). Die Nervenzellen werden über äußere Mikroelektroden gezielt stimuliert, die Signalübertragung bzw. Signalweiterleitung wird als Ausgangssignal in Form eines Transistorstroms analysiert. Es wurde inzwischen nachgewiesen, dass sich die Reize über die Kontaktstellen der Neuronen in beiden Richtungen weiterleiten lassen. In der vorgestellten "Silicon-Neuron-Junction"-Anordnung erfolgt zwar die Erfassung der Signalübertragung über die Membran der Nervenzelle, jedoch wird die Zelle hierfür mittels PCT (Patch Clamp Technic) kontaktiert, um so Potentialdifferenzen messen zu können. Eine Messung der Änderung des Membranpotentials im Außenraum der Zelle ist aufgrund der gewählten Messanordnung nicht möglich.

Es ist Aufgabe der vorliegenden Erfindung, ein Verfahren und eine Vorrichtung der eingangs genannten Art zur Verfügung zu stellen, mit denen unter weitgehender Vermeidung der dargestellten Nachteile des Standes der Technik durch Messung extrazellulärer Größen eine Reaktion von Rezeptorzellen auf Umgebungsänderungen festgestellt werden können.

Die Aufgabe wird durch ein Verfahren der eingangs genannten Art dadurch gelöst, dass ein ein Multipolfeld bildendes asymmetrisches extrazelluläres elektrisches Potential der mindestens einen Rezeptorzelle hergestellt und das Multipolfeld zur Feststellung der Zellreaktion herangezogen wird.

Insbesondere Dipolfelder, also Multipolfelder I. Ordnung, sind mittels geeigneter Signalwandlersysteme messtechnisch erfassbar. Änderungen im Multipolfeld - auch wenn diese zeitlich langsam auftreten - können somit zur Bestimmung von die Änderung bewirkenden Größen, z. B. dem Vorhandensein bestimmter Substanzen oder ihrer Konzentrationsänderung, genutzt werden. Da die Messung der Multipolfelder im Außenraum der Zelle stattfinden kann, ist eine Zerstörung der Zellmembran durch Einführen von Elektroden nicht notwendig.

Das erfindungsgemäße Verfahren kann auch so ausgeführt werden, dass mindestens eine gentechnisch veränderte Rezeptorzelle eingesetzt wird. Dabei ist es denkbar gentechnisch veränderte Rezeptorzellen gleichzeitig mit nicht gentechnisch veränderten Rezeptorzellen einzusetzen.

Weiterhin kann das erfindungsgemäße Verfahren auch so ausgeführt werden, dass die Asymmetrie im extrazellulären elektrischen Potential der mindestens einen auf Umgebungsänderungen reagierenden Rezeptorzelle durch Einbetten der mindestens einen Rezeptorzelle in ein ihre geometrische Form bestimmendes Einbett-Element erreicht wird. Die Rezeptorzellen sind in der Regel ohne äußere Einflussnahme weitgehend geometrisch symmetrisch aufgebaut, so dass sich auch die eine Potentialdifferenz zwischen Außen- und Innenraum bewirkenden Ladungen in der Regel symmetrisch um den Mittelpunkt der Zelle verteilen. Ein allein im Außenraum der Zelle messbares Multipolfeld entsteht bei einer symmetrisch aufgebauten Zelle somit nicht. Durch äußere Einflussnahme kann die geometrische Form der Rezeptorzelle verändert werden. Beim Einbetten in cin hierfür geeignetes, z. B. eine poröse Struktur aufweisendes Einbett-Elcment, wird die geometrische Form der Rezeptorzelle durch die Struktur des Einbett-Elements vorgegeben. Die Rezeptorzelle wird sich zumindest zum Teil an die Porenwände anschmiegen und hierdurch eine asymmetrische geometrische Form annehmen, die bei Anregung der Zelle zu einer asymmetrischen Verteilung der Ladungsträger im Innenraum und Außenraum der Zelle führen kann, wodurch eine Asymmetrie im extrazellulären elektrischen Potential und damit das Entstehen eines extrazellulären Multipolfeldes erreicht wird. Denkbar wäre es auch, die geometrische Form der Rezeptorzellen durch geeignete elektromagnetische Felder möglicherweise in Kombination mit einem geeigneten Einbetten zu beeinflussen.

Das erfindungsgemäße Messverfahren kann auch so ausgeführt werden, dass für die Detektion bestimmter Substanzen oder zur Messung der Substanzkonzentration oder ihrer Änderung in einem zu untersuchenden Medium die Asymmetrie im extrazellulären elektrischen Potential der mindestens einen Rezeptorzelle durch eine gerichtete Zufuhr des Mediums erreicht wird.

Bei einer gerichteten Zufuhr werden die Substanzen zunächst einen ersten Teilbereich einer Rezeptorzelle erreichen und, z. B. durch Aktivierung von Rezeptorproteinen, Veränderungen der elektrischen Ladungsverteilung bewirken, die sich nicht sofort symmetrisch ausbilden. Somit kann es auch hierdurch zu Asymmetrien im extrazellulären elektrischen Potential der Zelle kommen. Es kann auch vorteilhaft sein, wenn alternativ oder zusätzlich zur gerichteten Zufuhr das Messverfahren so ausgeführt wird, dass zur Erreichung der Asymmetrie im extrazellulären elektrischen Potential die Zufuhr des Mediums für mindestens einen Teilbereich der mindestens einen Rezeptorzelle blockiert wird. Die Blockade kann beispielsweise durch Abdecken eines Teils der Zellmembran erfolgen.

Weiterhin kann das erfindungsgemäße Messverfahren auch so ausgeführt werden, dass eine Vielzahl von Rezeptorzellen derart in einem Verband angeordnet werden, dass sich die Multipolfelder zumindest einer Teilanzahl der Rezeptorzellen additiv überlagern. Hierdurch kann die vorgenannte zweite Signalverstärkungskaskade ausgenutzt werden. Die Anordnung der Rezeptorzellen in einem Verband stellt eine Nachbildung der natürlichen Insektenantennen dar.

Die vorgenannte Aufgabe wird mit einer Vorrichtung der eingangs genannten Art dadurch gelöst, dass Asymmetrisierungsmittel zur Erzeugung einer Asymmetrie im extrazellulären elektrischen Potential der mindestens einen Rezeptorzelle vorgesehen sind.

Eine Variante der erfindungsgemäßen Vorrichtung ergibt sich aus Unteranspruch 8.

Im folgenden werden vorteilhafte Ausführungsbeispiele des erfindungsgemäßen Verfahrens vorgestellt.

Zum Erhalt geeigneter Rezeptorzellen lassen sich mittels molekularbiologischer Verfahren Zelllinien erzeugen, die stabil zwei Biomoleküle exprimieren: Rezeptorproteine und lonenkanäle. Rezeptorproteine dienen dazu, spezifische Liganden in einer Lösung oder in der Atmosphäre zu detektieren und in ein intrazelluläres biochemisches Signal umzuwandeln. Die Rezeptorproteine aktivieren in der Regel nachgeschaltete Zielenzyme und es kommt zum Anstieg oder Abfall in der Konzentration eines intrazellulären Botenstoffes, z.B. cyclo AMP, cyclo GMP, Phosphonositid oder Ca²⁺-Ionen. Diese Botenstoffe lösen in den Zellen eine Vielfalt kurz- oder langfristiger zellulärer Prozesse aus. Wenn man die Rezeptorproteine gleichzeitig mit lonenkanälen exprimiert, die durch diese Botenstoffe geöffnet werden, werden die Zellen bei Aktivierung der Rezeptorproteine elektrisch erregt. Das heißt, durch Stimulation mit extrazellulären Liganden ändert sich das Membranpotential, das durch geeignete Anbindung an einen Mikroelektronik-Signalwandler als Biosensorgröße verwendet werden kann. Als membranständige Rezeptorproteine können beispielsweise klonierte Dopamin-Rezeptoren aus Drosophila dienen. Diese Rezeptoren können z. B. an die Zielenzyme in HEK 293 oder COS-1-Zellen ankoppeln, was zu einem starken Anstieg der cyclo AMP-Konzentration führt. Als lonenkanäle lassen sich sogenannte cyclo AMP-/cyclo GMP-gesteuerte Kanäle verwenden, die direkt durch die Bindung von cyclo AMP oder cyclo GMP an eine Bindestelle im Kanalprotein geöffnet werden. Es können Zelllinien erzeugt werden, die beide Proteine - den Dopamin-Rezeptor und den Ionenkanal - exprimieren. Die Empfindlichkeit des Zellsystems, auf Änderungen in der Dopamin-Konzentration zu reagieren, hängt ab von:
1. der Dissoziationskonstante der Bindung zwischen Dopamin und Rezeptor,
2. der Belegungsdichte der Dopamin-Rezeptoren und der Kanäle in der Membran der Zelllinie,
3. der Kopplungseffizienz zwischen Rezeptorprotein und Effektorenzym (einem sog. G-Protein und einer Adenylatzyklase, die cyclo AMP synthetisiert) und schließlich von
4. der cyclo AMP-Empfindlichkeit des lonenkanals.

Mit Hilfe von gentechnisch erzeugten Mutanten kann die Dopamin- bzw. die cyclo AMP-Empfindlichkeit des Rezeptors bzw. des lonenkanals gesteigert werden. Die Expressionsdichte der Proteine auf der Zelloberfläche kann durch die Wahl geeigneter Vektoren positiv beeinflusst werden. Es ist weiterhin möglich, Zellen mit Duftstoff-Rezeptoren auszustatten, die auf bestimmte flüchtige Substanzen reagieren.

Für eine Messung, zum Beispiel von Duftstoff-Konzentrationen, werden geeignete Rezeptorzel-Icn in die Struktur eines Festkörpers eingebettet, wobei die Festkörperstrukturen den Zellen eine Änderung ihrer äußeren Form aufzwingen, und zwar derart asymmetrischer Natur, dass die extrazellulären und intrazellulären, die Potentialdifferenz zwischen dem Außenraum und dem Innenraum der Zelle bewirkenden Ladungen sich ebenfalls asymmetrisch verteilen und somit ein von außen messbares Multipolfeld erzeugt wird. Die Zelleinbettung kann z. B. in meso- oder makroporöse Siliziumsubstrate erfolgen, um so eine geometrisch stark anisotrope Umgebung mit inhomogener Feldverteilung zur Erzeugung extrazellulärer Multipolfelder zu benutzen. Die im Außenraum der Zelle auftretenden Multipolfelder sind messbar und somit ihre Veränderung aufgrund sich zeitlich verändernder Membranpotentiale feststellbar. In der Regel werden Dipolfelder, das sind Multipolfelder 1. Ordnung, zur Messung ausgenutzt. Die elektrische Ableitung der entsprechenden Signale kann z. B. über Impedanzspektroskopie, kapazitive oder induktive Ableitung erfolgen. Abhängig von der Art des Signalwandlers sind weiterhin amperometrische, voltametrische, potentiometrische, coulometrische sowie konduktometrische Ausleseverfahren möglich. Die Zellen können beispielsweise über dem Gate oder der ionen-sensitiven Schicht der Halbleiterstruktur oder einer Mikroelektrode positioniert werden, wobei die Zellen an der Oberfläche fest verankert sein sollten. Jede einzelne, in geeigneter Weise eingebettete Zelle kann im Prinzip separat ausgelesen werden.

In einer weiteren Ausführungsform des erfindungsgemäßen Verfahrens kann anstelle der eine Asymmetrie in der Ladungsverteilung erzeugenden Einbettung oder zusätzlich zu dieser eine gerichtete Zufuhr der zu detektierenden und/oder in ihrer Konzentration zu bestimmenden Substanzen erfolgen. Eine gerichtete Zufuhr kann dazu führen, dass diejenigen Rezeptorproteine zuerst aktiviert werden, die sich in einem ersten Bereich der Zelle befinden, der vom Zustrom der zu detektierenden Substanzen bzw. von der Konzentrationsänderung zuerst erfasst wird. Rezeptorproteine in eine dem Zustrom abgewandten Bereich der Zelle werden zeitlich versetzt aktiviert. Dieser zeitliche Verzug kann ebenfalls zu einer asymmetrischen Ladungsverteilung und damit zu einem im Außenraum der Zelle feststellbaren Multipolfeld führen. Selbstverständlich können auch Bereiche einer Zelle komplett für den Zustrom der zu detektierenden Substanz dauerhaft blockiert werden, wodurch ebenfalls die gewünschten Asymmetrien in der Ladungsverteilung entstehen. Auf diese Weise ist auch ohne eine die äußere Form der Rezeptorzelle verändernde Einbettung eine Asymmetrie und somit eine Messung extrazellulärer Potentialänderungen ohne Messung der Potentialdifferenz zwischen Außen- und Innenraum der Zelle möglich.

In einer weiteren Ausführungsform können Rezeptorzellen in Form von geordneten Verbänden auf geeignete Halbleiteroberflächen aufgebracht werden, wobei die Verbände eine parallele Orientierung erzeugter Multipolmomente begünstigt. Die Signale der einzelnen Zellen können sich dann zu einem höheren Summensignal überlagern, wenn die Orientierung der Zellen im Verband und/oder die Strömungsrichtung des reizstofftragenden Mediums optimiert und die Größe des Zellverbandes sowie die Abstände zwischen den einzelnen Zellen auf die Strömungsgeschwindigkeit des Mediums und die Antwort der Zellen abgestimmt sind. Dabei können die einzelnen Zellen durch geeignete Einbettung bereits asymmetrisch vorgeformt sein. Selbstverständlich ist es auch bei einem Zellverband möglich, Multipolfelder durch gerichteten Zustrom der Substanzen und/oder durch Blockierung von Teilbereichen der Zelle zu erreichen.

Das resultierende Messsignal eines homogenen Halbleiter-Zellverband-Hybrids kann separat abgegriffen werden, was bei einer Kombination mehrerer Halbleiter-Zellverband-Hybriden mit Zellen unterschiedlicher Antwortcharakteristik verschiedener Reizstoffe zu der Möglichkeit einer parallelen Erfassung mehrerer Reizstoffe sowie ganzer Reizstoffrnuster, dem sogenannten "Chemical Imaging", führen kann. Dieses Ausführungsbeispiel stellt eine halb-technische Nachbildung von Insektenantennen dar, mit der mittels extrazellulärer Ableitung über eine Halbleiterkomponente verletzungsfrei gemessen werden kann. In der Natur findet man in den entsprechenden Sinnesorganen häufiger röhren-, kamm- oder federähnliche Struktureinheiten, die bei einer technischen Realisierung als Muster dienen können.

Gegenüber den Inscktenantennen hat ein solches Hybridsystem den Vorteil, dass die überlagerten Signale nicht zwangsläufig von schwer kultivierbaren Nervenzellen stammen müssen, was die Standzeit eines solchen Hybridsystems wesentlich erhöhen kann. Des weiteren können über molekularbiologische Techniken erhaltene Zellen mit einem einzigen Rezeptorproteintyp und zugehörigem lonenkanal hintereinandergeschaltet werden, womit beide biologischen Signalverstärkungskaskaden ausgenutzt werden können. Mit solchen separat ansteuerbaren Rezeptorzellverbänden können dann Arrays mit separat ansprechbaren Rezeptorproteinen unterschiedlicher Spezifität konstruiert werden, die dann ebenfalls ein "Chemical Imaging" ermöglichen würden, ohne die biologische Verschlüsselung von Geruchsreizen entschlüsseln oder aufwendige Überlagerungstechniken anwenden zu müssen. Auch bei Zellverbänden ist es möglich, die einzelne Zelle separat auszulesen.

## Patentansprüche

1. Messverfahren unter Einsatz mindestens einer biologischen Rezeptorzelle, bei dem die Reaktion der Rezeptorzelle auf Umgebungsänderungen mittels mindestens eines technischen Signalwandlers festgestellt und einer Auswertung zugeführt wird,
**dadurch gekennzeichnet, dass**
ein Multipolfeld bildendes, asymmetrisches extrazelluläres elektrisches Potential der mindestens einen Rezeptorzelle hergestellt und
das Multipolfeld zur Feststellung der Zellreaktion herangezogen wird.

2. Messverfahren nach Anspruch 1, **dadurch gekennzeichnet, dass** mindestens eine gentechnisch veränderte Rezeptorzelle eingesetzt wird.

3. Messverfahren nach Anspruch 1 oder 2, **dadurch gekennzeichnet, dass** die Asymmetrie im extrazellulären elektrischen Potential der mindestens einen auf Umgebungsänderungen reagierenden Rezeptorzelle durch vorheriges Einbetten der mindestens einen Rezeptorzelle in ein ihre geometrische Form bestimmendes Einbett-Element erreicht wird.

4. Messverfahren nach Anspruch 1 oder 2, **dadurch gekennzeichnet, dass** für die Detektion bestimmter Substanzen oder zur Messung der Substanzkonzentration oder ihrer Änderung in einem zu untersuchenden Medium die Asymmetrie im extrazellulären elektrischen Potential der mindestens einen Rezeptorzelle durch eine gerichtete Zufuhr des Mediums erreicht wird.

5. Messverfahren nach Anspruch 1 oder 2, **dadurch gekennzeichnet, dass** für die Detektion bestimmter Substanzen oder zur Messung der Substanzkonzentration oder ihrer Änderung in einem zu untersuchenden Medium die Asymmetrie im extrazellulären elektrischen Potential durch eine Blockade der Zufuhr des Mediums für mindestens einen Teilbereich der mindestens einen Rezeptorzelle erreicht wird.

6. Messverfahren nach einem der Ansprüche 1 bis 5, **dadurch gekennzeichnet, dass** eine Vielzahl von Rezeptorzellen derart in einem Verband angeordnet werden, dass sich die extrazellulären Multipolfelder zumindest einer Teilanzahl der Rezeptorzellen additiv überlagern.

7. Zur Durchführung des Messverfahrens nach einem der Anspnche I bis 6 geeignete Vorrichtung, urnfassend Mittel zur Aufnahme von mindestens einer Rezeptorzelle, eine Signalwandlereinheit sowie Asymmetrisierungsmittel zur Erzeugung einer Asymmetrie im extrazellulären elektrischen Potential der mindestens einen Rezeptorzelle,
**dadurch gekennzeichnet, dass**
die Asymmetrisierungsmittel Mittel zur gerichteten Zufuhr eines eine zu detektierende oder in ihrer Konzentration oder Konzentrationsänderung zu bestimmende Substanz enthaltenden Mediums umfassen.

8. Zur Durchführung des Messverfahrens nach einem der Ansprüche 1 bis 6 geeignete Vorrichtung, umfassend Mittel zur Aufnahme von mindestens einer Rezeptorzelle, eine Signalwandlereinheit sowie Asymmetrisierungsmittel zur Erzeugung einer Asymmetrie im extrazellulären elektrischen Potential der mindestens einen Rezeptorzelle,
**dadurch gekennzeichnet, dass**
die Asymmetrisierungsmittel Mittel zur Blockade der Zufuhr eines eine zu detektierende oder in ihrer Konzentration oder Konzentrationsänderung zu bestimmende Substanz enthaltenden Mediums für mindestens einen Teilbereich der mindestens einen Rezeptorzelle umfassen.

## Claims

1. Measurement method using at least one biological receptor cell, wherein the reaction of the receptor cell to environmental changes is determined by means of at least one technical signal transducer and delivered for evaluation,
**characterized in that**
an asymmetric extracellular electrical potential of the at least one receptor cell is produced, which forms a multipole field, and
the multipole field is used to determine the cell reaction.

2. Measurement method according to Claim 1,
**characterized in that** at least one genetically modified receptor cell is used.

3. Measurement method according to Claim 1 or 2,
**characterized in that** the asymmetry in the extracellular electrical potential of the at least one receptor cell, which reacts to environmental changes, is achieved by previously embedding the at least one receptor cell in an embedding element which dictates its geometrical shape.

4. Measurement method according to Claim 1 or 2,
**characterized in that** for detecting particular substances, or for measuring the substance concentration or its change in a medium to be studied, the asymmetry in the extracellular electrical potential of the at least one receptor cell is achieved by directional delivery of the medium.

5. Measurement method according to Claim 1 or 2,
**characterized in that** for detecting particular substances, or for measuring the substance concentration or its change in a medium to be studied, the asymmetry in the extracellular electrical potential of the at least one receptor cell is achieved by a blockage of the delivery of the medium for at least a subregion of the at least one receptor cell.

6. Measurement method according to one of Claims 1 to 5, **characterized in that** a multiplicity of receptor cells are arranged in a group, so that the extracellular multipole fields of at least a subset of the receptor cells are additively superposed.

7. Device suitable for carrying out the measurement method according to one of Claims 1 to 6, comprising means for holding at least one receptor cell, a signal transducer unit and asymmetrization means for generating an asymmetry in the extracellular electrical potential of the at least one receptor cell,
**characterized in that**
the asymmetrization means comprise means for directional delivery of a medium containing a substance to be detected, or have its concentration or concentration change found.

8. Device suitable for carrying out the measurement method according to one of Claims 1 to 6, comprising means for holding at least one receptor cell, a signal transducer unit and asymmetrization means for generating an asymmetry in the extracellular electrical potential of the at least one receptor cell,
**characterized in that**
the asymmetrization means comprise means for blocking the delivery of a medium containing a substance to be detected, or have its concentration or concentration change found, for at least one subregion of the at least one receptor cell.

## Revendications

1. Procédé de mesure utilisant au moins une cellule réceptrice biologique, dans lequel la réaction de la cellule réceptrice aux modifications de l'environnement est détectée au moyen d'au moins un convertisseur de signaux technique et envoyée à l'exploitation,
**caractérisé en ce que**
un potentiel électrique extracellulaire asymétrique formant un champ multipolaire de l'au moins une cellule réceptrice est créé et
qu'on utilise le champ multipolaire pour détecter la réaction de la cellule.

2. Procédé de mesure selon la revendication 1, **caractérisé en ce qu'**au moins une cellule réceptrice modifiée par technique génétique est utilisée.

3. Procédé de mesure selon la revendication 1 ou 2, **caractérisé en ce que** l'asymétrie dans le potentiel électrique extracellulaire de l'au moins une cellule réceptrice réagissant à des modifications de l'environnement est obtenue par encastrement préalable de l'au moins une cellule réceptrice dans un élément d'encastrement définissant sa forme géométrique.

4. Procédé de mesure selon la revendication 1 ou 2, **caractérisé en ce que**, pour la détection de certaines substances ou pour la mesure de la concentration de substance ou de sa modification dans un milieu à analyser, l'asymétrie dans le potentiel électrique extracellulaire de l'au moins une cellule réceptrice est obtenue par apport dirigé du milieu.

5. Procédé de mesure selon la revendication 1 ou 2, **caractérisé en ce que**, pour la détection de certaines substances ou pour la mesure de la concentration de substance ou de sa modification dans un milieu à analyser, l'asymétrie dans le potentiel électrique extracellulaire est obtenue par blocage de l'apport de milieu pour au moins une zone partielle de l'au moins une cellule réceptrice.

6. Procédé de mesure selon l'une quelconque des revendications 1 à 5, **caractérisé en ce qu'**une multitude de cellules réceptrices sont agencées dans un groupe de manière à ce que les champs multipolaires extracellulaires se superposent de manière additive à au moins un nombre partiel de cellules réceptrices.

7. Dispositif adapté pour la réalisation du procédé de mesure selon l'une quelconque des revendications 1 à 6, comprenant des moyens destinés à recevoir au moins une cellule réceptrice, une unité de conversion de signaux ainsi que des moyens de création d'asymétrie pour générer une asymétrie dans le potentiel électrique extracellulaire de l'au moins une cellule réceptrice,
**caractérisé en ce que**
les moyens de création d'asymétrie comprennent des moyens d'apport dirigé d'un milieu contenant une substance à détecter ou à déterminer au niveau de sa concentration ou de sa modification de concentration.

8. Dispositif adapté pour la réalisation du procédé de mesure selon l'une quelconque des revendications 1 à 6, comprenant des moyens de réception d'au moins une cellule réceptrice, une unité de conversion de signaux ainsi que des moyens de création d'asymétrie pour générer une asymétrie dans le potentiel électrique extracellulaire de l'au moins une cellule réceptrice,
**caractérisé en ce que**
les moyens de création d'asymétrie comprennent des moyens de blocage de l'apport d'un milieu contenant une substance à détecter ou à déterminer au niveau de sa concentration ou de sa modification de concentration pour au moins une zone partielle de l'au moins une cellule réceptrice.
